# EUROPEAN PATENT SPECIFICATION

(11) **EP 3 818 533 B1**
(45) Date of publication and mention of the grant of the patent: **09.09.2026**
(21) Application number: 19765324.9
(22) Date of filing: 03.07.2019
(51) Int. Cl.: G16H 20/10, G16H 20/13, A61J 1/03, B65D 75/36, A61J 7/04, B65D 75/32

(54) **SMART MEDICATION BLISTER PACK**
INTELLIGENTE BLISTERPACKUNG FÜR MEDIKAMENTE
PLAQUETTE DE MÉDICAMENT INTELLIGENTE

(30) Priority: 03.07.2018 GB 201810881
(43) Date of publication of application: 12.05.2021
(73) Proprietor: Alertapack Ltd, Hove, Sussex BN3 7LS (GB)
(72) Inventor: VAN TENAC, Phillip John, Hove, Sussex BN3 7LS (GB)
(74) Representative: You Patent
(86) International application number: PCT/IB2019/055674
(87) International publication number: WO 2020/008385

(56) References cited:
- EP-A1- 3 269 345
- WO-A2-2014/088692
- US-A1- 2007 008 121
- US-A1- 2012 203 573
- US-A1- 2016 103 085
- US-B1- 8 960 440

## Description

### TECHNICAL FIELD

The presently disclosed subject matter generally relates to the field of medicine. Particularly, the present subject matter relates to a smart medication blister pack including one or more cavities of storing tablets, at least one of a chip for storing instructions, and a circuit, or a radio frequency identifier (RFID) tag.

### BACKGROUND

Millions of people in the world take medicines or vitamin supplements on daily basis for treating one or other diseases or for general wellness. Usually a doctor prescribes these medicines and provides instructions about time for taking the medicines to the patients verbally or in written. Sometimes, the patients have to take more than one medicine in a day at same or different times. Therefore, it becomes difficult for the patients to remember timings and instructions for taking each and every medicine. Also, taking medicines at wrong timing may result in health hazards for the patients.

Another problem these days in the world of medicines is counterfeiting of medicines. These days, most of the medicines available in the market are not authentic/genuine and its almost impossible for the patients to identify whether the medicine is authentic or fake. Consuming the fake medicine or drugs may be hazardous to the health of the patients. Prior arts US2016/103085A1 and US2012/203573A1 discussed the related technologies.

In light of above discussion, there exists need for improved techniques for informing the patients about the correct timing for taking the medication and also about counterfeiting of the medicines.

### SUMMARY

To overcome the problems mentioned above, the present disclosure provides a smart medication blister pack configured to receive instructions from an authorized person about tablets stored in the blister pack, and storing the instructions in a chip of the blister pack. The smart blister pack may remind a user like a patient about the timing, dosage etc. based on the stored instructions. Further, the smart blister pack may communicate with a central server to determine an authenticity of the tablets, and inform the user about the authenticity of the tablets accordingly.

The present disclosure provides a smart medication blister pack capable of storing one or more instructions or information. Further, the smart medication blister pack may be configured to arrange or modify the stored information and instructions based on one or more events for example, but not limited to, a time zone, a time when the pack breaks, and so forth. Further the smart medication blister pack is configured to communicate with a third party that can be an authorized person, a mobile device or a server.

The present disclosure provides a smart medication blister pack for packing of the medicine/tablets/pills. The blister pack comprises a chip and a circuitry comprising one or more modules. In some embodiments, the circuit includes at least one battery. The blister pack may comprise one or more light emitting diodes for reminding a patient about a time of taking the medicine. In alternative embodiments, the smart medication blister pack comprises a luminescent coating, ink or any material, which can be stuck, sprayed, printed onto the foil to emit light when its relevant circuit is activated. The luminescent coating may illuminate to remind a user about the stored medicine/tablets/pills. Further, a foil of the smart medication blister pack may be in a suitable form configured to be retrofittable to existing blister packs as at least one of a label or as an alternative foil of a suitable material.

The present disclosure provides a smart medication blister pack configured to receive instructions and store instructions in a cloud network. Further, the blister pack may be configured to update or change the instructions in real-time.

The present disclosure provides a smart medication blister pack for packing of the medicines in a secure manner. Further, the smart medication blister pack comprises a circuit comprising one or more modules. The smart medication blister pack can communicate or exchange information with a remote server via the circuit. Further, an authorized person like doctor, pharmacist, etc. can store instructions in the smart medication blister pack either indirectly via a remote server or a mobile phone/smart watch or directly.

The present disclosure provides a smart medication blister pack capable of storing information including but not limited to, batch number, date of manufacturing, dosage timing information, manufacturer information, uses, side effects, identification information and so forth.

The present disclosure provides a smart medication blister pack comprising a feedback module or a reminder module for providing a feedback or a reminder to a user such as, but not limited to, a patient. The feedback or the reminder may comprise at least one of an audio reminder, a video/visual reminder message, and a text message on an associated mobile device in communication with the smart medication blister pack. In some embodiments, the blister pack provides a visual indication based on light emitting diodes or the luminescent coating/ink on the circuit of the smart medication blister pack. Further, the smart medication blister pack may comprise batteries providing power to the LEDs. The batteries may be solar operated batteries.

The present disclosure provides a smart medication blister pack configured to program or re-program itself based on one or more events comprising such as, but not limited to, breakage of the blister pack, a timing the blister pack breaks, a location of the smart blister pack, and so forth. For example, the smart medication blister pack may adjust the timing of subsequent dosage as per the time of the dose taken recently. In some embodiments the smart medication blister pack is configured to arrange/change the stored instructions based on a time or time zone information where the smart medication blister pack is used. For example, if the smart blister pack is manufactured in United States of America (USA) so initially instructions are fed into it according to the time zone of USA. The smart medication bister pack can determine where the smart medication blister pack is and it's packing is breaking in which location or country. If the packing of the smart medication blister pack breaks in Australia then the smart medication blister pack may change or arrange pre-stored instructions according to the time zone of Australia. In some embodiments, the smart medication blister pack is configured to check location of the smart medication blister pack at regular intervals and may automatically arrange the instructions accordingly.

The present disclosure provides a smart medication blister pack configured to communicate the information to a central server so as to check whether the medicine in the smart medication blister pack is authentic or genuine or not.

In some embodiments, the cavities of the smart medication blister pack comprises a luminescent coating, ink or any material, which can be stuck, sprayed, printed onto the foil to emit light when its relevant circuit is activated. The luminescent coating may illuminate to remind a user about the stored medicine/tablets/pills.

The present disclosure provides a smart medication blister pack comprising a chip for storing information such as, but not limited to, batch number, manufacturer, company name, expiry date, manufacturing date, uses, dosage, time for taking medicine, side-effects, and so forth. An authorized person can feed instructions comprising the information in the chip.

The present disclosure provides a smart medication blister pack comprising a circuit having various modules. The smart medication blister pack is configured to receive instructions from a third party authorized to provide instructions and/or a central server.

The present disclosure provides a smart medication blister pack configured to remind patient about timings of taking the medicines. In some embodiments, the smart medication blister pack also reminds the patients in case patient takes more or less dosage of a particular medicine from the blister pack.

The present disclosure provides a smart medication blister pack comprising a plurality of cavities for storing medicines. In some embodiments, the blister pack may store more than one type of medicine in cavities. The blister pack may have a circuit embossed in a cover of the blister pack. The circuit may include a visual means for indicating or reminding the patient about time or dosage of the medicine. In some embodiments, the circuit includes LEDs and batteries for providing visual reminders to the patient. Further, the LEDs may light in different colors or patterns for providing different type of reminders to the patient.

The present disclosure provides a smart medication blister pack communicates with a central server when one or more events occur. The events may include, but not limited to, breakage of the blister pack. The central server in an embodiments may check if the medicine is genuine and authentic or not, and accordingly a feedback may be provided to the patient by the smart medication blister pack.

The present disclosure provides a smart medication blister pack configured to connect to a mobile phone of a user like a patient. In some embodiments, the smart medication blister pack may connect to the mobile phone via a mobile application. Further, the smart medication blister pack may provide reminders or the feedback to the patient on the mobile phone. The feedback may include at least one of an audio feedback, a video feedback, and a text feedback. The reminders may include at least one of an audio reminder, a video or visual reminder, and a text reminder.

An embodiment of the present disclosure provides a smart medication blister pack including a number of cavities configured to store one or more tablets, a cover, at least one of a circuit printed on the cover and a radio frequency identification (RFID) tag. The smart medication blister pack also includes a reminder module configured to provide a reminder to a user when at least one cavity of the cavities breaks. Further, the smart medication blister pack includes a receiving module configured to receive one or more instructions about the medication from an authorized person. Further, the smart medication blister pack includes a self-programming module configured to arrange the one or more instructions comprising one or more timing of the medication based on one or more events. Furthermore, the smart medication blister pack includes a communication module configured to communicate with one or more computing devices.

According to another aspect of the present disclosure, the cover may be formed using a suitable material such as, but not limited to, paper, plastic, laminate, metal for example aluminum, silver, etc., or any metallic foil.

According to another aspect of the present disclosure, the one or more computing devices comprising at least one of a mobile phone, a mobile phone application, a personal digital assistant, a smart watch, a fitness tracking device, a smart television, a tablet computer, a laptop, a computer, and a server.

According to another aspect of the present disclosure, the user receives a reminder on an associated computing device that is in communication with the smart medication blister pack, wherein the reminder being a medication reminder.

According to another aspect of the present disclosure, the smart medication blister pack further includes a chip configured to store the one or more instructions and information about the one or more tablets, a manufacturer of the medicine, dosage, diseases, side effects, usage, expiry date, manufacturing date, and batch information, wherein the communication module communicates the information with the one or more computing devices.

According to another aspect of the present disclosure, the communication module is further configured to send the cavity breakage information to a central server.

According to another aspect of the present disclosure, the reminder module further includes at least one of an audio module for providing an audio reminder and a visual module (or a visual module or a display module) for providing a visual reminder.

According to another aspect of the present disclosure, the reminder module is configured to inform the user if the one or more tablets is not authentic based on one or more communication with the central server received when the cavity breaks.

Another embodiment of the present disclosure provides a smart medication blister pack including a number of cavities configured to keep one or more tablets and a cover. The smart medication blister pack also includes a circuit printed on the cover and a radio frequency identification (RFID) tag. Further, the smart medication blister pack includes reminder module comprising at least one of an audio module and a visual module (hereinafter may also be referred as a video module or a display module) for providing an audio reminder and a video or visual reminder, respectively, to a user when at least one of the cavities breaks. The user receives a reminder on an associated computing device that is in communication with the smart medication blister pack. The smart medication blister pack also includes a receiving module configured to receive one or more instructions about the medication from an authorized person. Further, the smart medication blister pack includes a self-programming module configured to arrange the one or more instructions of the medication based on one or more events. Further, the smart medication blister pack includes a communication module configured to communicate with one or more computing devices. Furthermore, the smart medication blister pack includes a chip for storing the one or more instructions and information about the one or more tablets, a manufacturer of the medicine, dosage, diseases, side effects, usage, expiry date, manufacturing date, and batch information. The communication module communicates the information with the one or more computing devices.

A yet another embodiment of the present disclosure provides a smart medication blister pack including a plurality of cavities configured to keep one or more tablets, a cover, a circuit printed on the cover, and a radio frequency identification (RFID) tag. The smart medication blister pack also includes a reminder module comprising at least one of an audio module and a visual module (may be referred as a video module or a display module) for providing an audio reminder and a visual reminder, respectively to a user when at least one of the plurality of cavities breaks, wherein the user receives a reminder on an associated computing device that is in communication with the smart medication blister pack. The smart medication blister pack also includes a receiving module configured to receive one or more instructions about the medication from an authorized person. The smart medication blister pack further includes a self-programming module configured to arrange the one or more instructions of the medication based on one or more events. The smart medication blister pack further includes a communication module configured to communicate with one or more computing devices. The smart medication blister pack furthermore includes a chip for storing the one or more instructions and information about the one or more tablets, a manufacturer of the medicine, dosage, diseases, side effects, usage, expiry date, manufacturing date, and batch information, wherein the communication module communicates the information with the one or more computing devices. Further, the smart medication blister pack includes an authentication module configured to check if the smart medication blister pack is authentic based on at least one of information of the blister pack and communication with a server.

According to an aspect of the present disclosure, the reminder module is configured to inform the user if the blister pack is authentic or not based on the at least one of information of the smart medication blister pack and the communication with the server.

According to another aspect of the present disclosure, the smart medication blister pack further includes a battery configured to provide power to the circuit.

According to an aspect of the present disclosure, the one or more events includes at least one of a breaking of at least one of the plurality of cavity, a time at which the cavity breaks, and a time zone.

According to another aspect of the present disclosure, the one or more computing devices comprising at least one of a mobile phone, a mobile phone application, a personal digital assistant, a smart watch, a fitness tracking device, a smart television, a tablet computer, a laptop, a computer, and a server.

According to another aspect of the present disclosure, the authorized person includes at least one of a doctor, a patient, the user, a pharmacist, a manufacturer of the medication, and a caretaker.

According to another aspect of the present disclosure, the one or more instructions includes at least one of a timing for taking the one or more tablets, a date of manufacturing of the one or more tablets, a batch information of the one or more tablets, an expiry information of the one or more tablets, manufacturer information, and dosage information.

According to another aspect of the present disclosure, a foil of the smart medication blister pack may be in a suitable form configured to be retrofittable to existing blister packs as at least one of a label or as an alternative foil of a suitable material.

According to another aspect of the present disclosure, the reminder module is configured to inform the user if the one or more tablets is not authentic based on one or more communication with the central server received when the cavity breaks.

According to another aspect of the present disclosure, the communication module is further configured to send the cavity breakage information and information of the smart medication blister pack to a central server.

According to yet another aspect of the present disclosure, the smart medication blister pack further includes an authentication module configured to check if the smart medication blister pack is authentic based on at least one of information of the blister pack and communication with the central server.

According to another aspect of the present disclosure, the reminder module is configured to inform the user if at least one of the smart medication blister pack and the one or more tablets of the blister pack is authentic or not based on at least one of the communication with the central server and the information of the smart medication blister pack.

According to yet another aspect of the present disclosure, the smart medication blister pack further includes a ridge and notch for allowing a suitable device to slot and lock the smart medication blister pack into a position such that the circuit is lined up and make contact with the device.

The systems and methods disclosed in the present disclosure are widely applicable to the field of patient management, medicines, and hospitals.

Other and further aspects and features of the disclosure will be evident from reading the following detailed description of the embodiments, which are intended to illustrate, not limit, the present disclosure.

### BRIEF DESCRIPTION OF THE DRAWINGS

The illustrated embodiments of the disclosed subject matter will be best understood by reference to the drawings, wherein like parts are designated by like numerals throughout. The following description is intended only by way of example, and simply illustrates certain selected embodiments of devices, systems, and processes that are consistent with the disclosed subject matter as claimed herein.
FIG. 1 is a schematic diagram illustrating an exemplary environment, where various embodiments of the present disclosure may function; and
FIG. 2 is a block diagram illustrating various system elements of an exemplary smart medication blister pack, in accordance with an embodiment of the present disclosure.

### DETAILED DESCRIPTION

The following detailed description is made with reference to the figures. Exemplary embodiments are described to illustrate the disclosure, not to limit its scope, which is defined by the claims. Those of ordinary skill in the art will recognize a number of equivalent variations in the description that follows.

The functional units described in this specification have been labeled as devices. A device may be implemented in programmable hardware devices such as processors, digital signal processors, central processing units, field programmable gate arrays, programmable array logic, programmable logic devices, cloud processing systems, or the like. The devices may also be implemented in software for execution by various types of processors. An identified device may include executable code and may, for instance, comprise one or more physical or logical blocks of computer instructions, which may, for instance, be organized as an object, procedure, function, or other construct. Nevertheless, the executable of an identified device need not be physically located together, but may comprise disparate instructions stored in different locations which, when joined logically together, comprise the device and achieve the stated purpose of the device.

Indeed, an executable code of a device or module could be a single instruction, or many instructions, and may even be distributed over several different code segments, among different applications, and across several memory devices. Similarly, operational data may be identified and illustrated herein within the device, and may be embodied in any suitable form and organized within any suitable type of data structure. The operational data may be collected as a single data set, or may be distributed over different locations including over different storage devices, and may exist, at least partially, as electronic signals on a system or network.

Reference throughout this specification to "a select embodiment," "one embodiment," or "an embodiment" means that a particular feature, structure, or characteristic described in connection with the embodiment is included in at least one embodiment of the disclosed subject matter. Thus, appearances of the phrases "a select embodiment," "in one embodiment," or "in an embodiment" in various places throughout this specification are not necessarily referring to the same embodiment.

Furthermore, the described features, structures, or characteristics may be combined in any suitable manner in one or more embodiments. In the following description, numerous specific details are provided, to provide a thorough understanding of embodiments of the disclosed subject matter. One skilled in the relevant art will recognize, however, that the disclosed subject matter can be practiced without one or more of the specific details, or with other methods, components, materials, etc. In other instances, well-known structures, materials, or operations are not shown or described in detail to avoid obscuring aspects of the disclosed subject matter.

The smart medication blister pack comprises a circuit that may include a software, hardware, firmware, or combination of these.

In accordance with the exemplary embodiments, the disclosed computer programs or modules can be executed in many exemplary ways, such as an application that is resident in the memory of a device or as a hosted application that is being executed on a server and communicating with the device application or browser via a number of standard protocols, such as TCP/IP, HTTP, XML, SOAP, REST, JSON and other sufficient protocols. The disclosed computer programs can be written in exemplary programming languages that execute from memory on the device or from a hosted server, such as BASIC, COBOL, C, C++, Java, Pascal, or scripting languages such as JavaScript, Python, Ruby, PHP, Perl or other sufficient programming languages.

Some of the disclosed embodiments include or otherwise involve data transfer over a network, such as communicating various inputs or files over the network. The network may include, for example, one or more of the Internet, Wide Area Networks (WANs), Local Area Networks (LANs), analog or digital wired and wireless telephone networks (e.g., a PSTN, Integrated Services Digital Network (ISDN), a cellular network, and Digital Subscriber Line (xDSL)), radio, television, cable, satellite, and/or any other delivery or tunneling mechanism for carrying data. The network may include multiple networks or sub networks, each of which may include, for example, a wired or wireless data pathway. The network may include a circuit-switched voice network, a packet-switched data network, or any other network able to carry electronic communications. For example, the network may include networks based on the Internet protocol (IP) or asynchronous transfer mode (ATM), and may support voice using, for example, VoIP, Voice-over-ATM, or other comparable protocols used for voice data communications. In one implementation, the network includes a cellular telephone network configured to enable exchange of text or SMS messages.

Examples of the network include, but are not limited to, a personal area network (PAN), a storage area network (SAN), a home area network (HAN), a campus area network (CAN), a local area network (LAN), a wide area network (WAN), a metropolitan area network (MAN), a virtual private network (VPN), an enterprise private network (EPN), Internet, a global area network (GAN), and so forth.

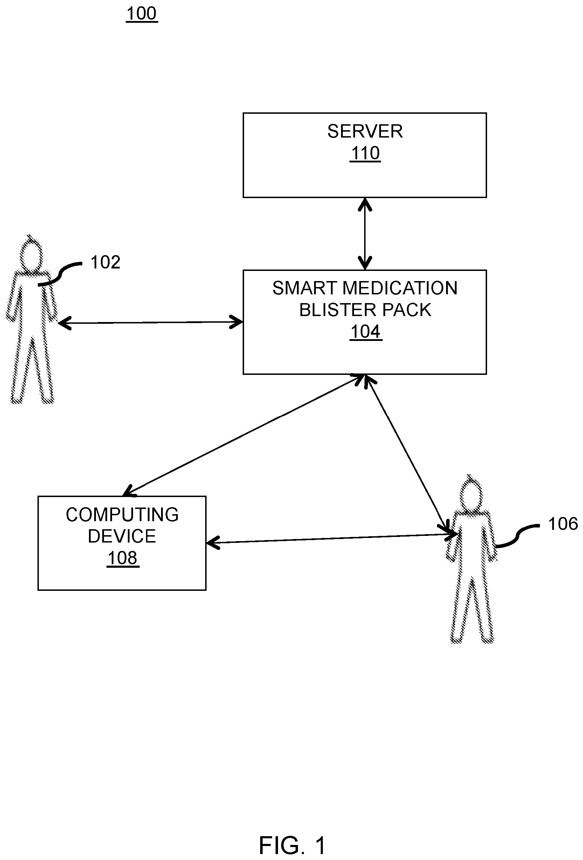

FIG. 1 is a schematic diagram illustrating an exemplary environment 100, where various embodiments of the present disclosure may function. As shown, the environment 100 includes a user 102, an authorized person 106 having an associated computing device 108, a smart medication blister pack 104, and a server 110. The user 102 may be a patient. The computing device 108 can be any suitable device such as, but not limited to, a smart phone, a desktop computer, a laptop computer, a smart watch, a fitness tracking device, a tablet computer, a mobile phone, a personal digital assistant, the server 110, a smart television (TV), and so forth, capable of communicating with the smart medication blister pack 104. In some embodiments, the computing device 108 communicates with the smart medication blister pack 104 via one or more mobile applications.

The smart medication blister pack 104 includes a number of cavities configured to store one or more tablets. The smart medication blister pack also includes a cover. The cavities may store the medicine, pills, capsules, or the one or more tablets. The smart medication blister pack 104 may pack the medicines in a secure manner. In some embodiments, the smart medication blister pack 104 (hereinafter also referred as a blister pack 104) may store more than one type of medicine or tablet in one or more of the cavities.

Further, the smart medication blister pack 104 includes at least one of a circuit printed on the cover and a radio frequency identification (RFID) tag. In some embodiments of the present disclosure, the cover may be formed using a suitable material such as, but not limited to, paper, plastic, laminate, metal for example aluminum, silver, etc., or any metallic foil.

In some embodiments, the smart medication blister pack 104 may have a circuit embossed in the cover of the blister pack 104. In some embodiments, the circuit may include a visual means for indicating or reminding the patient (i.e. the user 102) about time or dosage of the medicine. In some embodiments, the circuit includes LEDs and batteries for providing visual reminders to the patient. Further, the LEDs may light in different colors or patterns for providing different type of reminders to the user 102. The smart medication blister pack 104 also includes a battery. The battery may be a source of energy configured to provide power, for example a push - a voltage - of energy, to get the current flowing in the circuit of the smart medication blister pack 104. In some embodiments, the smart medication blister pack 104 may include a radio frequency identification (RFID) tag.

Further, the smart medication blister pack 104 includes a reminder module configured to provide a reminder to a user when at least one cavity of the cavities breaks. The reminder module may further include at least one of an audio module for providing an audio reminder and a visual module for providing a visual reminder. The user 102 may receive a reminder on the associated computing device 108 that is in communication with the smart medication blister pack. The reminder may be a medication reminder. Further, the reminder module may be configured to inform the user 102 if the one or more tablets is not authentic based on one or more communication with the central server 110 received when the cavity breaks.

The smart medication blister pack 104 further includes a receiving module configured to receive one or more instructions about the medication from the authorized person 106. The authorized person 106 can be any person authorized to feed or store instructions in the smart medication blister pack 104. Examples of the authorized person 106 may include, but are not limited to a doctor, the user 102, a manufacturer of the one or more tablets, a caretaker, a pharmacist, a patient, and so forth. Examples of the one or more instructions may include, but are not limited to, a timing for taking the one or more tablets, a date of manufacturing of the one or more tablets, a batch information of the one or more tablets, an expiry information of the one or more tablets, manufacturer information, and dosage information. In some embodiments, the authorized person 106 provides instructions comprising a timing for taking the tablets, a date of manufacturing of the tablets, a batch information of the tablets, an expiry information of the tablets, manufacturer information, and so forth.

The smart medication blister pack 104 also includes a self-programming module configured to arrange the one or more instructions comprising one or more timing of the medication based on one or more events. The one or more events may include such as, but not limited to, a breaking of at least one of the plurality of cavity, a time at which the cavity breaks, and a time zone.

The smart medication blister pack 104 also includes a communication module configured to communicate with one or more computing devices such as, the computing device 108. In some embodiments, the communication module is further configured to send the cavity breakage information to a central server 110.

The smart medication blister pack 104 may also include a chip to store the one or more instructions and information about the one or more tablets, a manufacturer of the medicine, dosage, diseases, side effects, usage, expiry date, manufacturing date, batch information, and so forth. The communication module communicates the information with the one or more computing devices.

The server 110 may be a remotely located central server storing and maintaining information about the medicines stored in the smart medication blister pack 104. The information may include, but are not limited to, batch number, date of expiry, date of manufacturing, dosage information, manufacturer of the medicine, side effects, usage, identification, and so forth. The information may include all the information required to determine an authenticity of medicines including the one or more tablets stored in the blister pack 104. The central server 110 may also include a database of information about a number of medicines.

In some embodiments, the smart medication blister pack 104 may include an RFID tag, a circuit and/or a chip. The smart medication blister pack 104 may also include the circuit including one or more modules. The circuit may include at least one battery. The smart medication blister pack 104 may include one or more light emitting diodes (LEDs) for reminding the user 102 about a time of taking the medicine i.e. the one or more tablets stored in the smart medication blister pack 104.

Further, the smart medication blister pack 104 may include one or more LEDs. The smart medication blister pack 104 may communicate with the server 110 and the computing device 108 via the circuit. Further, the smart medication blister pack 104 may receive one or more instructions from the authorized person 106 and/or the server 110 directly or indirectly over a network (not shown). In some embodiments, the smart medication blister pack 104 may be configured to receive instructions and store instructions in a cloud network. Further, the smart medication blister pack 104 may be configured to automatically update or change the instructions in real-time.

The chip of the smart medication blister pack 104 may store the one or more instructions. The LEDs may light up based on the instructions. Further, the smart medication blister pack 104 may be configured to arrange or modify the stored information and instructions based on one or more events for example, but not limited to, a time zone, a time when the pack breaks, and so forth.

In alternative embodiments, the smart medication blister pack 104 may include a luminescent coating, ink or any material, which can be stuck, sprayed, printed onto the foil to emit light when its relevant circuit is activated. The luminescent coating may illuminate to remind a user about the stored medicine/tablets/pills. The luminescent coating may be on each of the cavities or only on some of the cavities. In alternative embodiments, the luminescent coating may be on the circuit of the smart medication blister pack 104.

Further the smart medication blister pack 104 may provide a feedback or a reminder to a user such as, but not limited to, a patient. The feedback or the reminder may comprise at least one of an audio reminder, a visual reminder (or a video reminder) message, and a text message on the associated mobile/computing device 108 in communication with the smart medication blister pack. In some embodiments, the blister pack104 may provide a visual indication based on light emitting diodes on the circuit of the blister pack 104. Further, the smart medication blister pack 104 may comprise batteries for providing power to the LEDs. In some embodiments, the batteries may be solar operated batteries.

Furthermore, the smart medication blister pack 104 may be configured to program or re-program itself based on one or more events including such as, but not limited to, breakage of the blister pack, a timing the blister pack breaks, a location of the blister pack 104, and so forth. For example, the smart medication blister pack 104 may adjust the timing of subsequent dosage as per the time of the dose taken recently. In some embodiments the smart medication blister pack 104 is configured to arrange/change the stored instructions based on a time or time zone information where the smart medication blister pack is used. For example, if the smart blister pack 104 is manufactured in India so initially instructions are fed into it according to the time zone of India. The smart medication bister pack 104 can determine where the smart medication blister pack 104 is and it's packing is breaking in which location or country. If the packing of the smart medication blister pack 104 breaks in USA then the smart medication blister pack 104 may change or arrange pre-stored instructions according to the time zone of USA. In some embodiments, the smart medication blister pack 104 is configured to check location of the smart medication blister pack 104 at regular intervals and may automatically arrange the stored instructions accordingly.

Further, the smart medication blister pack 104 may be configured to communicate the information to the server 110 so as to check whether the medicine/pills/tablets/capsule stored in the smart medication blister pack 104 is authentic or genuine or fake.

Further, the smart medication blister pack 104 may be configured to remind patient about timings of taking the medicines. In some embodiments, the smart medication blister pack 104 also reminds the patients or the user 102 in case the user 102 takes more or less dosage of a particular medicine (i.e. the one or more tablets) from the smart medication blister pack 104.

Further, the smart medication blister pack 104 may be configured to connect to the computing device 108 such as, a mobile phone of the user 102 such as, a patient. Hereinafter, the user 102 may be referred as a patient 102, without change in its meaning. Further, hereinafter, the computing device 108 may also be referred as the mobile phone 108. In some embodiments, the smart medication blister pack 104 may connect to the mobile phone via a mobile application. Further, the smart medication blister pack 104 may provide reminders or the feedback to the patient 102 on the mobile phone 108. The feedback may include at least one of an audio feedback, a video feedback, a visual notification, and a text feedback. The reminders may include at least one of an audio reminder, a video or visual reminder, and a text reminder.

In some embodiments of the present disclosure, the smart medication blister pack 104 further includes a roll of foil with an RFID chip, substrate circuits covering each component (or cavity) and a printed coating configured to be illuminated once a circuit is powered to indicate the compartment to open. The smart medication blister pack 104 further includes one or more circuit connectors to connect to one or more connectors of a suitable device.

In some embodiments, the foil of the smart medication blister pack 104 may be in a suitable form configured to be retrofittable to existing blister packs as either a label or as an alternative foil of a suitable material.

Further, in some embodiments of the present disclosure, the smart medication blister pack 104 further includes a ridge and notch for allowing a suitable device to slot and lock the smart medication blister pack 104 into a position such that the circuit is lined up and make contact with the device.

In some embodiments, the blister pack 104 may store pre-stored information and/or instructions about the medicine (i.e. the one or more tablets) and the user 102 or the authorized person 106 may not be required to feed or store information or instructions in the blister pack 104.

Further, the smart medication blister pack 104 may communicate with the central server 110 when one or more events occur. In some embodiments, the smart medication blister pack communicates with the central server 110 over a wireless network by using wireless communication technologies like Wi-Fi, Bluetooth, Near Field Communication (NFC), and so forth. In some embodiments, the central server 110 may check if the medicine is genuine and authentic or not, and accordingly send a notification to the smart medication blister pack 104. The smart medication blister pack 104 may then accordingly inform the user 102 and may also provide a feedback about the authenticity of the one or more tablets contained in the blister pack 104 to the user 102. This way the smart medication blister pack 104 may prevent the user 102 from consuming fake and wrong medicines.

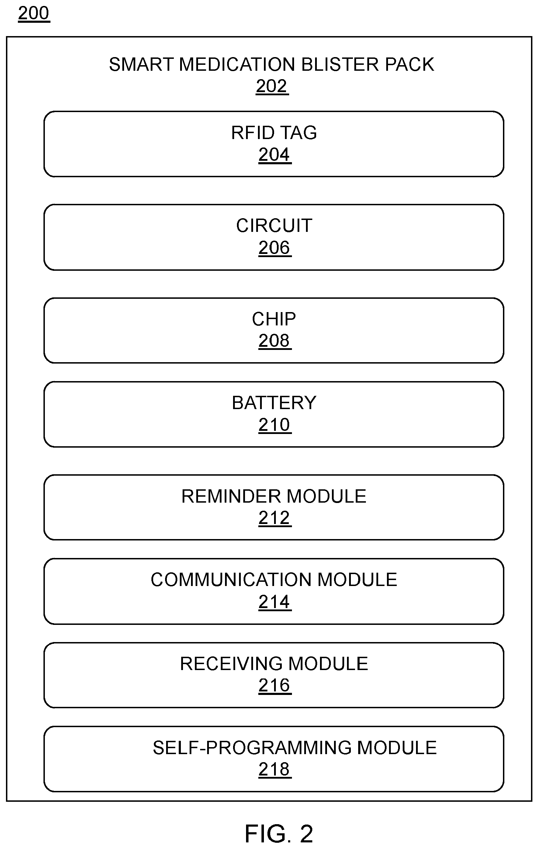

FIG. 2 is a block diagram 200 illustrating various system elements of an exemplary smart medication blister pack 202, in accordance with an embodiment of the present disclosure. As shown, the smart medication blister pack 202 primarily includes an RFID tag 204 storing the identification information of the medication or tablets stored in the smart medication blister pack 202, a circuit 206, a chip 208, at least one battery 210, a reminder module 212, a communication module 214, a receiving module 216, and a self-programming module 218. Though not shown, but the smart medication blister pack 202 may include less or more number of modules.

The RFID tag 204 may electronically store one or more information about the medicines stored in the smart medication blister pack 202. In some embodiments, the information stored in the RFID tag 204 may be read using an RFID reader device. In some embodiments, the circuit 206 includes the modules 208-218. The circuit 206 may be printed or embossed on the cover of the smart medication blister pack 202. The smart medication blister pack 202 may further include one or more cavities for storing the medicine, pills or tablets. In some embodiments, the smart medication blister pack 202 (hereinafter also referred as a blister pack 202) may store more than one type of medicine in the cavities. In some embodiments, the circuit 206 may include a visual means for indicating or reminding the patient (i.e. the user 102) about time or dosage of the medicine. As discussed with reference to FIG. 1, in some embodiments, the circuit 206 includes LEDs and batteries for providing visual reminders to the user 102 for example, a patient. Further, the LEDs may light in different colors or patterns for providing different type of reminders to the patient.

In some embodiments of the present disclosure, the smart medication blister pack 202 further includes a ridge and notch for allowing a suitable device to slot and lock the smart medication blister pack 202 into a position such that the circuit 206 is lined up and make contact with the device.

The chip 208 may store one or more instructions or information about the stored medicine/pills/tablets, a manufacturer of the medicine, dosage, diseases, side effects, usage, expiry date, manufacturing date, batch information, and so forth. An authorized person like the authorized person 106 may provide the instructions or the information. The authorized person can be any person authorized to feed or store instructions in the smart medication blister pack 202. In some embodiments, the authorized person 106 provides instructions such as, but not limited to, a timing for taking the tablets, a date of manufacturing of the tablets, a batch information of the tablets, an expiry information of the tablets, manufacturer information, and so forth.

Further, the smart medication blister pack 202 may include one or more LEDs. The smart medication blister pack 202 may communicate with the server 110 and the computing device 108 via the circuit 206. In some embodiments, the smart medication blister pack 104 may store the one or more instructions in the chip 208. In some embodiments, the LEDs may light up based on the instructions.

The reminder module 212 is configured to provide a reminder to a user when at least one cavity of the cavities breaks. The reminder module 212 may further include at least one of an audio module for providing an audio reminder and a video module (hereinafter may also be referred as a visual module or a display module) for providing a visual/video reminder to a user when at least one of the cavities breaks. The visual module may include one or more light emitting diodes (LEDs). The LEDs may light up based on the instructions. Further, the LEDs may light in different colors or patterns for providing different type of reminders to the patient i.e. the user 102. The audio module may include a speaker. The audio and visual modules may provide a feedback to the patient or the user 102. In some embodiments, the patient receives a reminder on the mobile phone in communication with the smart medication blister pack 202. Further, the reminder module 212 is configured to inform the patient if the medicine is not authentic based on one or more communication with the central server 110 that may be received when the cavity breaks. The feedback or the reminder may include at least one of an audio reminder, a video/visual reminder message, and a text message on the associated mobile/computing device 108 in communication with the smart medication blister pack 202. In some embodiments, the smart medication blister pack 202 may provide a visual indication based on light emitting diodes on the circuit 206 of the blister pack 202. Further, the smart medication blister pack 104 may include the at least one battery 210 for providing power to the LEDs. In some embodiments, the battery 210 (or batteries) may be solar operated battery. The at least one battery 210 may provide power to the LEDs and the battery 210 may be a solar battery.

The communication module 214 may be configured to communicate with one or more device such as, the server 110 and the computing device 108 as discussed with reference to FIG. 1. Further, the one or more computing devices may include, but are not limited to, a mobile phone, a mobile phone application, a personal digital assistant, and so forth. Further, the communication module 214 may send the cavity breakage information to the central server 110. Further, the communication module 214 may receive one or more instructions from the authorized person 106 and/or the server 110 directly or indirectly over a network (not shown).

In some embodiments, when one or more events occur, the smart medication blister pack 104 may communicate with the central server 110 (or computing device, or web-based or cloud based device) via a wireless communication network using wireless communication technologies like NFC, Wi-Fi, Bluetooth, etc.

The smart medication blister pack 202 may further include pre-stored instructions or information about the one or more tablets. The smart medication blister pack 104 may be configured to update the pre-stored instructions or information automatically for example by communicating with the central server 110 or other computing devices.

The receiving module 216 may be configured to receive one or more instruction about the medication from the authorized person 106. The authorized person 106 may include such as, but not limited to, a doctor, a patient, a pharmacist, a manufacturer of the medication, and a caretaker. Further, the authorized person 106 may provide instructions to the smart medication blister pack 202. The instructions may include such as, but not limited to, a timing for taking the tablets, a date of manufacturing of the tablets, a batch information of the tablets, an expiry information of the tablets, manufacturer information, a dosage information, and so forth. The communication module 214 may communicate the information with the one or more computing devices.

The self-programming module 218 may be configured to arrange one or more instructions, such as one or more timing of the medication, based on one or more events. The one or more events may include such as, but not limited to, a breaking of at least one of the plurality of cavity, a time at which the cavity breaks, and a time zone. The self-programming module 218 may be configured to automatically update or change the instructions in real-time.

The disclosed smart medication blister pack have cavities to keep the tablets and each of the cavities have visual means to inform the patient or the user about medication timing reminder.

In alternative embodiments, the smart medication blister pack comprises a luminescent coating, ink or any material, which can be stuck, sprayed, printed onto the foil to emit light when its relevant circuit is activated. The luminescent coating may illuminate to remind a user about the stored medicine/tablets/pills. The luminescent coating may be on each of the cavities or only on some of the cavities. In alternative embodiments, the luminescent coating may be on the circuit of the smart medication blister pack.

In some embodiments, the foil of the smart medication blister pack 202 may be in a suitable form configured to be retrofittable to existing blister packs as either a label or as an alternative foil of a suitable material.

In some embodiments, the communication module 214 is further configured to send the cavity breakage information and information of the smart medication blister pack 202 to a central server like the server 110.

In some embodiments, the smart medication blister pack 202 of claim 8 further comprising an authentication module (not shown) configured to check if the smart medication blister pack is authentic based on at least one of information of the blister pack 202 and communication with the server 110

The reminder module 212 may be configured to inform the user 102 if at least one of the smart medication blister pack 202 and the one or more tablets of the blister pack 202 is authentic or not based on at least one of the communication with the central server 110 and the information of the smart medication blister pack 202.

In an exemplary scenario, when the timing of taking a medicine happens, one of the cavities of the smart medicine blister pack may illuminate to remind the user to take that medicine in the cavity. Further, the user may receive a reminder message on his/her associated device like mobile phone, smart watch etc.

In another exemplary scenario, when a user/patient breaks a cavity of the smart blister pack for example for the first time, the smart medication blister pack may communicate with the server to check an authenticity of the contained medicine. Further, the user/patient may be notified about the authenticity of the medicine via an audio means or/and visual means.

The disclosed smart medication blister pack may also have audio means to inform the patient about the medication timing reminder.

The disclosed smart medication blister pack may also have a chip for storing the instructions and information about the medication.

The present disclosure provides a smart medication blister pack including a plurality of cavities for keeping one or more tablets and a cover; at least one of a circuit printed on the cover and a radio frequency identification (RFID) tag; a reminder module having at least one of an audio means and a video means for providing a reminder to a user when at least one of the plurality of cavities breaks; a self-programming module configured to arrange one or more timing of the medication based on one or more events; a communication module configured to communicate with one or more device; and an instruction receiving module configured to receive one or more instruction about the medication from an authorized person. The cover may be formed using a suitable material such as, but not limited to, paper, plastic, laminate, metal for example aluminum, silver, etc., or any metallic foil.

The disclosed smart medication blister pack is capable of storing one or more instructions or information. Further, the smart medication blister pack may be configured to arrange or modify the stored information and instructions based on one or more events for example, but not limited to, a time zone, a time when the pack breaks, and so forth. Further the smart medication blister pack is configured to communicate with a third party that can be an authorized person, a mobile device or a server.

The disclosed smart medication blister pack is for packing of the medicine/tablets/pills. The smart medication blister pack includes a chip and a circuit including one or more modules. In some embodiments, the circuit includes at least one battery. The smart medication blister pack may comprise one or more light emitting diodes for reminding a patient about a time of taking the medicine. In alternative embodiments, the smart medication blister pack includes a luminescent coating, ink or any material, which can be stuck, sprayed, printed onto the foil to emit light when its relevant circuit is activated. The luminescent coating may illuminate to remind a user about the stored medicine/tablets/pills.

The disclosed smart medication blister pack is configured to receive instructions and store instructions in a cloud network. Further, the smart medication blister pack may be configured to update or change the instructions in real-time.

The disclosed smart medication blister pack may pack and store the medicines in a secure manner. Further, the smart medication blister pack comprises a circuit comprising one or more modules. The smart medication blister pack can communicate or exchange information with a remote server via the circuit. Further, an authorized person like doctor, pharmacist, etc. can store instructions in the smart medication blister pack either indirectly via a remote server or a mobile phone/smart watch or directly.

The disclosed smart medication blister pack is capable of storing information including but not limited to, batch number, date of manufacturing, dosage timing information, manufacturer information, uses, side effects, identification information, and so forth.

The disclosed smart medication blister pack includes a feedback module or a reminder module for providing a feedback or a reminder to a user such as, but not limited to, a patient. The feedback or the reminder may include at least one of an audio reminder, a video/visual reminder message, and a text message on an associated mobile or the computing device in communication with the smart medication blister pack. In some embodiments, the blister pack provides a visual indication based on light emitting diodes or the luminescent coating/ink on the circuit of the smart medication blister pack. Further, the smart medication blister pack may comprise batteries providing power to the LEDs. The batteries may be solar operated batteries.

The disclosed smart medication blister pack may program or re-program itself based on one or more events comprising such as, but not limited to, breakage of the blister pack, a timing the blister pack breaks, a location of the smart blister pack, and so forth. For example, the smart medication blister pack may adjust the timing of subsequent dosage as per the time of the dose taken recently. In some embodiments the smart medication blister pack is configured to arrange/change the stored instructions based on a time or time zone information where the smart medication blister pack is used. For example, if the smart blister pack is manufactured in United States of America (USA) so initially instructions are fed into it according to the time zone of USA. The smart medication bister pack can determine where the smart medication blister pack is and it's packing is breaking in which location or country. If the packing of the smart medication blister pack breaks in Australia then the smart medication blister pack may change or arrange pre-stored instructions according to the time zone of Australia. In some embodiments, the smart medication blister pack is configured to check location of the smart medication blister pack at regular intervals and may automatically arrange the instructions accordingly.

The disclosed smart medication blister pack may communicate the information to a central server so as to check whether the medicine in the smart medication blister pack is authentic or genuine or not.

In some embodiments, the cavities of the smart medication blister pack includes a luminescent coating, ink or any material, which can be stuck, sprayed, printed onto the foil to emit light when its relevant circuit is activated. The luminescent coating may illuminate to remind a user about the stored medicine/tablets/pills.

The disclosed smart medication blister pack includes a chip for storing information such as, but not limited to, batch number, manufacturer, company name, expiry date, manufacturing date, uses, dosage, time for taking medicine, side-effects, and so forth. An authorized person can feed instructions comprising the information in the chip.

The disclosed smart medication blister pack includes a circuit having various modules. The smart medication blister pack is configured to receive instructions from a third party authorized to provide instructions and/or a central server.

The disclosed smart medication blister pack is configured to remind patient about timings of taking the medicines. In some embodiments, the smart medication blister pack also reminds the patients in case patient takes more or less dosage of a particular medicine from the blister pack.

The disclosed smart medication blister pack includes a plurality of cavities for storing medicines. In some embodiments, the blister pack may store more than one type of medicine in cavities. The blister pack may have a circuit embossed in a cover of the smart medication blister pack. The circuit may include a visual means for indicating or reminding the patient about time or dosage of the medicine. In some embodiments, the circuit includes LEDs and batteries for providing visual reminders to the patient. Further, the LEDs may light in different colors or patterns for providing different type of reminders to the patient.

According to an aspect of the present disclosure, the cover may be formed using a suitable material such as, but not limited to, paper, plastic, laminate, metal for example aluminum, silver, etc., or any metallic foil.

The disclosed smart medication blister pack communicates with a central server when one or more events occur. The events may include, but not limited to, breakage of the blister pack. The central server in some- embodiments may check if the medicine is genuine and authentic or not, and accordingly a feedback may be provided to the patient by the smart medication blister pack.

The disclosed a smart medication blister pack configured to connect to a mobile phone of a user like a patient. In some embodiments, the smart medication blister pack may connect to the mobile phone via a mobile application running on the mobile phone. Further, the smart medication blister pack may provide reminders or the feedback to the patient on the mobile phone. The feedback may include at least one of an audio feedback, a notification, a video feedback, and a text feedback. The reminders may include at least one of an audio reminder, a video/visual reminder, and a text reminder.

It will be understood that the devices and the databases referred to in the previous sections are not necessarily utilized together method or system of the embodiments. Rather, these devices are merely exemplary of the various devices that may be implemented within a computing device or the server device, and can be implemented in exemplary another devices, and other devices as appropriate, that can communicate via a network to the exemplary server device.

It will be appreciated that several of the above-disclosed and other features and functions, or alternatives thereof, may be desirably combined into many other different systems or applications. Various presently unforeseen or unanticipated alternatives, modifications, variations, or improvements therein may be subsequently made by those skilled in the art, which are also intended to be encompassed by the following claims.

The above description does not provide specific details of manufacture or design of the various components. Those of skill in the art are familiar with such details, and unless departures from those techniques are set out, techniques, known, related art or later developed designs and materials should be employed. Those in the art are capable of choosing suitable manufacturing and design details.

The terminology used herein is for the purpose of describing particular embodiments only and is not intended to be limiting of the disclosure. It will be appreciated that several of the above-disclosed and other features and functions, or alternatives thereof, may be combined into other systems, methods, or applications. Various presently unforeseen or unanticipated alternatives, modifications, variations, or improvements therein may subsequently be made by those skilled in the art without departing from the scope of the present disclosure as encompassed by the following claims.

## Claims

1. A smart medication blister pack (104, 202) comprising:
a plurality of cavities configured to store one or more tablets;
a cover;
at least one of a circuit (206) printed on the cover and a radio frequency identification (RFID) tag (204);
a reminder module (212) configured to provide a reminder to a user when at least one cavity of the plurality of cavities breaks;
a receiving module (216) configured to receive one or more instructions about the medication from an authorized person;
a self-programming module (218) configured to arrange the one or more instructions comprising one or more timing of the medication based on one or more events; and
a communication module (214) configured to communicate with one or more computing devices, the communication module (214) is further configured to send the cavity breakage information and information of the smart medication blister pack to a central server (110),
further comprising an authentication module configured to check if the smart medication blister pack is authentic based on at least one of information of the blister pack and communication with the central server (110),
**characterized in that** the reminder module (212) is configured to inform the user if at least one of the smart medication blister pack and the one or more tablets of the blister pack is authentic or not based on at least one of the communication with the central server (110) and the information of the smart medication blister pack.

2. The smart medication blister pack (104, 202) of claim 1, wherein the one or more events comprising at least one of a breaking of at least one of the plurality of cavity, a time at which the cavity breaks, and a time zone, further wherein the one or more computing devices (108) comprising at least one of a mobile phone, a mobile phone application, a personal digital assistant, a smart watch, a fitness tracking device, a smart television, a tablet computer, a laptop, a computer, and a server.

3. The smart medication blister pack (104, 202) of claim 2, wherein the user (102) receives a reminder on an associated computing device (108) that is in communication with the smart medication blister pack (104, 202), wherein the reminder being a medication reminder.

4. The smart medication blister pack (104, 202) of claim 1, wherein the authorized person (106) comprising at least one of a doctor, a patient, the user, a pharmacist, a manufacturer of the one or more tablets, and a caretaker, wherein the one or more instructions comprising at least one of a timing for taking the one or more tablets, a date of manufacturing of the one or more tablets, a batch information of the one or more tablets, an expiry information of the one or more tablets, manufacturer information, and dosage information.

5. The smart medication blister pack (104, 202) of claim 4 further comprising a chip configured to store the one or more instructions and information about the one or more tablets, a manufacturer of the medicine, dosage, diseases, side effects, usage, expiry date, manufacturing date, and batch information, wherein the communication module (214) communicates the information with the one or more computing devices (108).

6. The smart medication blister pack (104, 202) of claim 1, wherein the reminder module (212) further comprising at least one of an audio module for providing an audio reminder and a visual module for providing a visual reminder.

7. The smart medication blister pack (104, 202) of claim 1 further comprising a battery (210) configured to provide power to the circuit (206).

8. The smart medication blister pack (104, 202) of claim 1, wherein the cover is formed using a material comprising at least one of paper, plastic, laminate, metal, and any metallic foil.

9. The smart medication blister pack (104, 202) of claim 1 further comprising a ridge and notch for allowing a suitable device to slot and lock the smart medication blister pack (104, 202) into a position such that the circuit is lined up and make contact with the device.

10. The smart medication blister pack (104, 202) of claim 1 further comprising a foil of the smart medication blister pack (104, 202) may be in a suitable form configured to be retrofittable to existing blister packs as at least one of a label or as an alternative foil of a suitable material.

## Patentansprüche

1. Eine intelligente Medikamenten-Blisterverpackung (104, 202), umfassend:
eine Vielzahl von Vertiefungen, die zur Aufnahme einer oder
mehrerer Tabletten ausgebildet sind; eine Abdeckung;
mindestens entweder eine auf den Deckel aufgedruckte Schaltung (206) oder einen RFID-Tag (204);
ein Erinnerungsmodul (212), das so konfiguriert ist, dass es einen Benutzer erinnert, wenn mindestens eine der mehreren Vertiefungen bricht;
ein Empfangsmodul (216), das so konfiguriert ist, dass es eine oder mehrere Anweisungen bezüglich des Medikaments von einer autorisierten Person empfängt;
ein selbstprogrammierendes Modul (218), das so konfiguriert ist, dass es die eine oder mehrere Anweisungen, die einen oder mehrere Zeitpunkte der Medikamenteneinnahme umfassen, auf der Grundlage eines oder mehrerer Ereignisse anordnet; und
ein Kommunikationsmodul (214), das so konfiguriert ist, dass es mit einem oder mehreren Computergeräten kommuniziert; wobei das Kommunikationsmodul (214) ferner so konfiguriert ist, dass es die Informationen über den Bruch der Vertiefung und Informationen über die intelligente Medikamentenblisterpackung an einen zentralen Server (110) sendet,
das ferner ein Authentifizierungsmodul umfasst, das so konfiguriert ist, dass es auf der Grundlage mindestens einer der Informationen der Blisterpackung und der Kommunikation mit dem zentralen Server (110) prüft, ob die intelligente Medikamenten-Blisterpackung authentisch ist,
**dadurch gekennzeichnet, dass** das Erinnerungsmodul (212) so konfiguriert ist, dass es den Benutzer darüber informiert, ob mindestens eines der intelligenten Medikamenten-Blisterpackungen und die eine oder mehreren Tabletten der Blisterpackung echt sind oder nicht, basierend auf mindestens einem der folgenden Elemente: der Kommunikation mit dem zentralen Server (110) und den Informationen der intelligenten Medikamenten-Blisterpackung.

2. Die intelligente Medikamentenblisterpackung (104, 202) nach Anspruch 1, wobei das eine oder die mehreren Ereignisse mindestens eines der folgenden Elemente umfassen: ein Aufbrechen mindestens einer der mehreren Vertiefungen, einen Zeitpunkt, zu dem die Vertiefung aufbricht, und eine Zeitzone, wobei ferner das eine oder die mehreren Computergeräten (108) mindestens eines der folgenden Elemente umfassen: ein Smartphone, eine Smartphone-App, einen Personal Digital Assistant, eine Smartwatch, ein Fitness-Tracking-Gerät, einen Smart-TV, einen Tablet-Computer, einen Laptop, einen Computer und einen Server umfassen.

3. Die intelligente Medikamenten-Blisterpackung (104, 202) nach Anspruch 2, wobei der Benutzer (102) eine Erinnerung auf einem zugehörigen Comput ergerät(108) erhält, das mit der intelligenten Medikamenten-Blisterpackung (104, 202) in Verbindung steht, wobei die Erinnerung eine Medikamentenerinnerung ist.

4. Die intelligente Medikamentenblisterpackung (104, 202) nach Anspruch 1, wobei die autorisierte Person (106) mindestens eine der folgenden Personen umfasst: einen Arzt, einen Patienten, den Benutzer, einen Apotheker, einen Hersteller der einen oder mehreren Tabletten und eine Pflegekraft, wobei die eine oder mehreren Anweisungen mindestens eines der folgenden Elemente umfassen: einen Zeitpunkt für die Einnahme der einen oder mehreren Tabletten, ein Herstellungsdatum der einen oder mehreren Tabletten, eine Chargeninformation der einen oder mehreren Tabletten, eine Verfallsinformation der einen oder mehreren Tabletten, Herstellerinformationen und Dosierungsinformationen.

5. Die intelligente Medikamenten-Blisterpackung (104, 202) nach Anspruch 4, die ferner einen Chip umfasst, der so konfiguriert ist, dass er die eine oder mehrere Anweisungen und Informationen über die eine oder mehreren Tabletten, den Hersteller des Arzneimittels, die Dosierung, Krankheiten, Nebenwirkungen, die Anwendung, das Verfallsdatum, das Herstellungsdatum und Chargeninformationen speichert, wobei das Kommunikationsmodul (214) die Informationen an die eine oder mehreren Computergeräten(108) übermittelt.

6. Die intelligente Medikamenten-Blisterpackung (104, 202) nach Anspruch 1, wobei das Erinnerungsmodul (212) ferner mindestens eines von einem Audiomodul zur Bereitstellung einer akustischen Erinnerung und einem visuellen Modul zur Bereitstellung einer visuellen Erinnerung umfasst.

7. Die intelligente Medikamenten-Blisterpackung (104, 202) nach Anspruch 1, die ferner eine Batterie (210) umfasst, die so konfiguriert ist, dass sie die Schaltung (206) mit Strom versorgt.

8. Die intelligente Medikamenten-Blisterpackung (104, 202) nach Anspruch 1, wobei die Abdeckung aus einem Material gebildet ist, das mindestens eines der folgenden umfasst: Papier, Kunststoff, Laminat, Metall und eine beliebige Metallfolie.

9. The smart medication blister pack (104, 202) of claim 1 further comprising a ridge and notch for allowing a suitable device to slot and lock the smart medication blister pack (104, 202) into a position such that the circuit is lined up and make contact with the device.

10. Die intelligente Medikamenten-Blisterverpackung (104, 202) nach Anspruch 1, die ferner eine Folie der intelligente Medikamenten-Blisterverpackung (104, 202) umfasst, kann in einer geeigneten Form vorliegen, die so ausgelegt ist, dass sie als Etikett oder als alternative Folie aus einem geeigneten Material nachträglich an bestehende Blisterverpackungen angebracht werden kann.

## Revendications

1. Une plaquette alvéolée intelligente de médicament (104, 202) comprenant :
une pluralité de cavités configurées pour stocker un ou
plusieurs comprimés ; un couvercle ;
au moins l'un d'un circuit (206) imprimé sur le couvercle et d'une étiquette d'identification par radiofréquence (RFID) (204) ;
un module de rappel (212) configuré pour fournir un rappel à un utilisateur lorsqu'au moins une cavité de la pluralité de cavités se rompt ;
un module de réception (216) configuré pour recevoir une ou plusieurs instructions concernant le médicament d'une personne autorisée ;
un module d'auto-programmation (218) configuré pour organiser la ou les instructions comprenant un ou plusieurs horaires du médicament sur la base d'un ou plusieurs événements ; et
un module de communication (214) configuré pour communiquer avec un ou plusieurs dispositifs informatiques, le module de communication (214) étant en outre configuré pour envoyer les informations de rupture de cavité et les informations de la plaquette alvéolée intelligente de médicament à un serveur central (110),
comprenant en outre un module d'authentification configuré pour vérifier si la plaquette alvéolée intelligente de médicament est authentique sur la base d'au moins l'un d'informations de la plaquette alvéolée et de la communication avec le serveur central (110),
**caractérisée en ce que** le module de rappel (212) est configuré pour informer l'utilisateur si au moins l'un de la plaquette alvéolée intelligente de médicament et du ou des comprimés de la plaquette alvéolée est authentique ou non sur la base d'au moins l'un de la communication avec le serveur central (110) et des informations de la plaquette alvéolée intelligente de médicament.

2. La plaquette alvéolée intelligente de médicament (104, 202) selon la revendication 1, dans laquelle le ou les événements comprennent au moins l'un d'une rupture d'au moins l'une de la pluralité de cavités, d'un moment auquel la cavité se rompt, et d'un fuseau horaire, dans laquelle en outre le ou les dispositifs informatiques (108) comprennent au moins l'un d'un téléphone mobile, d'une application de téléphone mobile, d'un assistant numérique personnel, d'une montre intelligente, d'un dispositif de suivi de la condition physique, d'un téléviseur intelligent, d'une tablette, d'un ordinateur portable, d'un ordinateur, et d'un serveur.

3. La plaquette alvéolée intelligente de médicament (104, 202) selon la revendication 2, dans laquelle l'utilisateur (102) reçoit un rappel sur un dispositif informatique associé (108) qui est en communication avec la plaquette alvéolée intelligente de médicament (104, 202), dans laquelle le rappel est un rappel de médicament.

4. La plaquette alvéolée intelligente de médicament (104, 202) selon la revendication 1, dans laquelle la personne autorisée (106) comprend au moins l'un d'un médecin, d'un patient, de l'utilisateur, d'un pharmacien, d'un fabricant du ou des comprimés, et d'un soignant, dans laquelle la ou les instructions comprennent au moins l'un d'un horaire pour la prise du ou des comprimés, d'une date de fabrication du ou des comprimés, d'informations de lot du ou des comprimés, d'informations de péremption du ou des comprimés, d'informations sur le fabricant, et d'informations de posologie.

5. La plaquette alvéolée intelligente de médicament (104, 202) selon la revendication 4 comprenant en outre une puce configurée pour stocker la ou les instructions et des informations concernant le ou les comprimés, un fabricant du médicament, la posologie, les maladies, les effets secondaires, l'utilisation, la date de péremption, la date de fabrication, et les informations de lot, dans laquelle le module de communication (214) communique les informations avec le ou les dispositifs informatiques (108).

6. La plaquette alvéolée intelligente de médicament (104, 202) selon la revendication 1, dans laquelle le module de rappel (212) comprend en outre au moins l'un d'un module audio pour fournir un rappel audio et d'un module visuel pour fournir un rappel visuel.

7. La plaquette alvéolée intelligente de médicament (104, 202) selon la revendication 1 comprenant en outre une batterie (210) configurée pour fournir de l'alimentation au circuit (206).

8. La plaquette alvéolée intelligente de médicament (104, 202) selon la revendication 1, dans laquelle le couvercle est formé en utilisant un matériau comprenant au moins l'un de papier, de plastique, de laminé, de métal, et de toute feuille métallique.

9. La plaquette alvéolée intelligente de médicament (104, 202) selon la revendication 1 comprenant en outre une nervure et une encoche pour permettre à un dispositif approprié d'emboîter et de verrouiller la plaquette alvéolée intelligente de médicament (104, 202) dans une position telle que le circuit soit aligné et entre en contact avec le dispositif.

10. La plaquette alvéolée intelligente de médicament (104, 202) selon la revendication 1 comprenant en outre une feuille de la plaquette alvéolée intelligente de médicament (104, 202) qui peut être sous une forme appropriée configurée pour être adaptable aux plaquettes alvéolées existantes en tant qu'au moins l'un d'une étiquette ou d'une feuille alternative d'un matériau approprié.
